# FASCICULE DE BREVET EUROPEEN

(11) **EP 3 893 796 B1**
(45) Date de publication et mention de la délivrance du brevet: **31.08.2022**
(21) Numéro de dépôt: 19839380.3
(22) Date de dépôt: 11.12.2019
(51) Int. Cl.: A61B 34/30, A61B 90/50

(54) **RECALAGE AUTOMATIQUE D'UN BRAS ROBOT POUR UNE INTERVENTION MÉDICALE**
AUTOMATISCHE REGISTRIERUNG EINES ROBOTERARMS FÜR EINEN MEDIZINISCHEN EINGRIFF
AUTOMATIC REGISTRATION OF A ROBOT ARM FOR A MEDICAL INTERVENTION

(30) Priorité: 12.12.2018 FR 1872735
(43) Date de publication de la demande: 20.10.2021
(73) Titulaire: Quantum Surgical, 34000 Montpellier (FR)
(72) Inventeur: NAHUM, Bertin, 34170 Castelnau-le-Lez (FR); BADANO, Fernand, 69006 LYON (FR); BLONDEL, Lucien, 34070 MONTPELLIER (FR); BANEGAS, Frédéric, 34830 JACOU (FR); OLIVE, Sébastien, 34000 MONTPELLIER (FR)
(74) Mandataire: Ipside
(86) Numéro de dépôt international: PCT/FR2019/053012
(87) Numéro de publication internationale: WO 2020/120901

(56) Documents cités:
- WO-A1-2018/167246
- US-A1- 2016 258 782
- US-A1- 2017 202 629
- NIXON ET AL: "THE EFFECTS OF METALS AND INTERFERING FILEDS ON ELECTROMAGNETIC TRACKERS", PRESENCE: TELEOPERATORS AND VIRTUAL ENVIRONMENTS, MIT PRES, CAMBRIDGE, MA, US, vol. 7, 1 janvier 1998 (1998-01-01), pages 204-218, XP008071806, ISSN: 1531-3263, DOI: 10.1162/105474698565587
- HUMMEL JOHANN ET AL: "Evaluation of a miniature electromagnetic position tracker", MEDICAL PHYSICS, AIP, MELVILLE, NY, US, vol. 29, no. 10, 1 octobre 2002 (2002-10-01), pages 2205-2212, XP012011606, ISSN: 0094-2405, DOI: 10.1118/1.1508377
- Julian Much: "Error Classification and Propagation for Electromagnetic Tracking", , 14 janvier 2008 (2008-01-14), XP055621337, Technical University of Munich, Munich, Germany Extrait de l'Internet: URL:http://campar.in.tum.de/twiki/pub/Stud ents/DaElMagError/DAMuch.pdf [extrait le 2019-09-11]

## Description

### Domaine de l'invention

La présente invention appartient au domaine des robots médicaux destinés à assister un praticien lors d'une intervention médicale. Notamment, l'invention concerne un robot médical assisté par un système de navigation électromagnétique et configuré pour positionner un bras articulé du robot médical dans une zone de mesure du système de navigation électromagnétique sans perturber, ou alors seulement de façon négligeable, les mesures réalisées par le système de navigation électromagnétique. L'invention concerne également un procédé de positionnement d'un bras articulé d'un tel robot médical.

### Etat de la technique

De nombreuses interventions médicales nécessitent de positionner ou de déplacer de manière très précise un instrument médical (par exemple une aiguille, un cathéter, une électrode, un générateur d'ultrasons, un foret de perçage, etc.) par rapport à une anatomie d'intérêt d'un patient.

Pour certaines interventions, il existe actuellement des robots médicaux permettant d'assister un praticien pour placer, maintenir ou guider un instrument médical.

Un tel robot médical doit être positionné de sorte à permettre ensuite au praticien d'exécuter un plan d'intervention comportant une ou plusieurs actions à effectuer sur une anatomie d'intérêt d'un patient.

Un tel plan d'intervention est généralement déterminé par le praticien, puis il est transmis au robot médical qui le mémorise dans une mémoire d'une unité de contrôle intégrée au robot médical et configurée pour diriger le robot médical.

Il convient alors, pour positionner le robot médical de façon optimale pour permettre au praticien d'exécuter les actions du plan d'intervention, de pouvoir définir précisément la position de l'anatomie d'intérêt par rapport au robot médical pendant l'intervention médicale.

Dans certains systèmes, et notamment dans le système décrit dans le brevet US 8774901 B2, la position de l'anatomie d'intérêt du patient peut être définie dans un repère du robot médical à l'aide d'une image intra-opératoire prise pendant l'intervention médicale. Dans un tel cas, la position du dispositif d'imagerie par rapport au robot médical doit être connue et fixée. L'image intra-opératoire permet en effet de déterminer la position de l'anatomie d'intérêt du patient par rapport au dispositif d'imagerie. Comme la position du dispositif d'imagerie par rapport au robot médical est fixe et connue, il est alors possible de déterminer la position de l'anatomie d'intérêt du patient par rapport au robot médical.

Avec un tel système, un déplacement de l'anatomie d'intérêt du patient pendant l'intervention médicale ne pourra pas être détectée sans qu'une autre image intra-opératoire ne soit prise. Un déplacement de l'anatomie d'intérêt peut être dû, par exemple, aux mouvements engendrés par la respiration du patient, aux mouvements des organes internes, ou aux manipulations intra-opératoires d'un opérateur sur le patient.

Dans un tel système, le robot médical est associé à un dispositif d'imagerie particulier. Le système est généralement fixé au sol d'une salle d'intervention. Le robot médical ne peut donc pas être utilisé avec un autre dispositif d'imagerie, ou bien dans une autre salle d'intervention sans une mise en place complexe.

En outre, un tel système nécessite une détermination du plan d'intervention au moment de l'intervention, à partir de l'image intra-opératoire. Les conditions de planification ne sont donc pas optimales pour le praticien qui a peu de temps pour définir les actions à effectuer sur l'anatomie d'intérêt.

Dans d'autres systèmes, et notamment dans le système décrit dans la demande de brevet US 2016/0157887 A1, la position de l'anatomie d'intérêt du patient peut être définie par rapport au robot médical à l'aide d'un système de navigation optique. Un capteur optique est placé à proximité de l'anatomie d'intérêt du patient. Un autre capteur optique est placé sur le robot médical. Le système de navigation optique permet de déterminer la position d'un capteur par rapport à l'autre. Un dispositif d'imagerie médicale est également utilisé pour déterminer la position que doit prendre un outil médical par rapport à l'anatomie d'intérêt dans un repère du capteur optique placé au niveau de l'anatomie d'intérêt du patient. La position de l'outil médical peut alors être déterminée dans un repère du robot médical.

Un tel système permet de suivre d'éventuels mouvements de l'anatomie d'intérêt pendant l'intervention, notamment les mouvements dus à la respiration du patient ou à des manipulations du patient par un opérateur pendant l'intervention.

Toutefois, un tel système n'est pas adapté pour déterminer précisément la position d'une anatomie d'intérêt située à l'intérieur du corps d'un patient. En effet, un système de navigation optique nécessite que les capteurs optiques soient visibles, avec une ligne de vue directe, par un dispositif de contrôle du système de navigation optique.

Aussi, tout objet venant interrompre la ligne de vue entre un capteur optique et le dispositif de contrôle du système de navigation optique pendant l'intervention aurait pour conséquence de causer des erreurs sur l'estimation de la position du robot médical par rapport à l'anatomie d'intérêt du patient, et donc sur le positionnement de l'outil médical par rapport à ladite anatomie d'intérêt.

Pour éviter d'avoir à garantir une ligne de vue directe entre un dispositif de contrôle et un capteur du système de navigation, il est envisageable de remplacer le système de navigation optique par un système de navigation électromagnétique.

Les systèmes de navigation électromagnétique présentent cependant l'inconvénient d'être sensibles aux interférences et aux distorsions du champ électromagnétique en présence d'objets métalliques. Il convient alors, par exemple, que seuls des matériaux non métalliques pénètrent dans la zone de mesure du système de navigation. Dans certains cas, des plaques d'aluminium sont utilisées pour isoler une partie métallique du robot médical par rapport à un générateur de champ électromagnétique du système de navigation.

Un moteur d'une articulation d'un bras articulé d'un robot médical comporte généralement des parties métalliques. Une réduction du nombre de parties métalliques, ou l'utilisation de plaques d'aluminium dans le bras articulé d'un robot médical entraîne donc une réduction des degrés de liberté du bras articulé. Autrement dit, le nombre de mouvements possibles du bras articulé est nettement réduit. Il peut alors devenir impossible de configurer le bras articulé dans une position permettant au praticien de réaliser avec suffisamment de précision certaines actions sur une anatomie d'intérêt d'un patient.

Le document « The effects of metals and interfering fields on electromagnetic trackers » de Nixon et al. s'intéresse à l'influence d'un élément métallique sur des mesures effectuées par un capteur d'un système de navigation électromagnétique.

Le document « Evaluation of a miniature electromagnetic position tracker » de J. Hummel et al. met en évidence l'influence de plusieurs éléments métalliques (guide-outil, instrument médical, etc.) sur la précision des mesures effectuées par un capteur d'un système de navigation électromagnétique utilisé dans des applications cliniques.

Le document « Error classification and propagation for electromagnetic tracking » de J. Much étudie et classifie différents types d'erreurs de mesure d'un capteur d'un système de navigation électromagnétique dans le cadre d'interventions chirurgicales assistées.

La demande de brevet US 2016/258782 A1 divulgue une méthode de compensation des erreurs de mesure d'un capteur d'un système de navigation électromagnétique engendrées par une distorsion de champ électromagnétique due à la présence d'un élément métallique à proximité du capteur.

La demande de brevet WO2018/167246 A1 divulgue une méthode de détermination de position d'un robot par un capteur électromagnétique d'un système de navigation électromagnétique. Des erreurs de mesure due à la présence d'un objet métallique à proximité du capteur sont réduites en positionnant le capteur proche du générateur de champ électromagnétique.

### Exposé de l'invention

La présente invention a pour objectif de remédier à tout ou partie des inconvénients de l'art antérieur, notamment ceux exposés ci-avant.

A cet effet, et selon un premier aspect, il est proposé par la présente invention un procédé de positionnement d'un bras articulé d'un robot médical pour assister un praticien lors d'une intervention médicale sur une anatomie d'intérêt d'un patient. Le robot médical comporte une base à laquelle est relié le bras articulé, ainsi qu'une unité de contrôle permettant de configurer le bras articulé dans une position déterminée. Le robot médical est assisté par un système de navigation électromagnétique comportant un générateur de champ électromagnétique et deux capteurs électromagnétiques. Le champ électromagnétique généré forme une zone de mesure dans laquelle la position d'un capteur peut être déterminée par le système de navigation électromagnétique et communiquée au robot médical. Un premier capteur est positionné, dans une étape préalable, au niveau de l'anatomie d'intérêt, un deuxième capteur est positionné sur le bras articulé. Le procédé de positionnement du bras articulé est remarquable en ce qu'il comporte les étapes suivantes :
- une détermination, lorsque les deux capteurs se trouvent dans la zone de mesure, d'une région dite « d'influence réduite », à partir de la position du générateur de champ électromagnétique et à partir de la position des capteurs,
- une configuration du bras articulé de telle sorte que toute partie métallique du bras articulé se trouvant dans la zone de mesure est située à l'intérieur de la région d'influence réduite.

Le procédé de positionnement du bras articulé ne comporte pas d'étape d'exécution d'un geste médical sur le patient. Le bras articulé du robot médical est en effet configuré préalablement à l'exécution du geste médical par le praticien.

Dans la présente demande, le terme « médical » est à considérer au sens large et peut concerner aussi bien un contexte chirurgical qu'un contexte non chirurgical. Par « anatomie d'intérêt » du patient, on entend une partie au moins du corps humain du patient pour laquelle un traitement est visé par l'intervention médicale. Dans la présente demande, le terme « position » correspond en fait à la combinaison de la position et de l'orientation d'un objet dans un repère donné qui est généralement un système de coordonnées en trois dimensions. Le terme « pose » est employé dans la littérature anglo-saxonne pour représenter cette combinaison de la position et de l'orientation d'un objet dans l'espace.

Le deuxième capteur positionné sur le bras articulé du robot médical est placé à une position connue dans un repère du robot médical.

Le système de navigation électromagnétique permet de déterminer la position du premier capteur et du deuxième capteur dans un repère du système de navigation électromagnétique. Un tel repère est par exemple centré par rapport à un centre du générateur de champ électromagnétique dudit système de navigation électromagnétique. La position du premier capteur et la position du deuxième capteur sont donc connues l'une par rapport à l'autre. Comme la position du deuxième capteur est connue dans un repère du robot médical, la position du premier capteur peut être déterminée dans un repère du robot médical. La position des capteurs et la position du générateur de champ électromagnétique dans le repère du système de navigation sont communiquées au robot médical par le système de navigation. Le terme « position » comprend la position et l'orientation dans les trois dimensions spatiales du repère du système de navigation.

La position de l'anatomie d'intérêt du patient est connue par rapport à la position du premier capteur. Par exemple, une image intra-opératoire sur laquelle apparaît à la fois l'anatomie d'intérêt du patient et la position du premier capteur peut être réalisée pendant l'intervention médicale.

Connaissant la position de l'anatomie d'intérêt par rapport au premier capteur et la position du premier capteur par rapport au robot médical, il est possible de déterminer la position de l'anatomie d'intérêt par rapport robot médical.

La région dite « d'influence réduite » est une région de la zone de mesure dans laquelle l'introduction d'un objet métallique ne perturbe quasiment pas la détermination des positions du premier capteur et du deuxième capteur par le système de navigation électromagnétique lorsque lesdits capteurs se trouvent dans la zone de mesure du champ électromagnétique généré par le générateur du système de navigation électromagnétique. Cette région est telle qu'un objet métallique situé dans cette région ne créé pas, ou alors de façon négligeable, de distorsions sur une ligne du champ électromagnétique généré passant par un des capteurs.

Il est convenu dans la présente demande que la perturbation sur les mesures de position des capteurs induite par la présence de parties métalliques du bras 14 articulé du robot médical 10 dans la zone d'influence réduite est négligeable si l'erreur de mesure de la position d'un capteur sur chaque axe d'un système de coordonnées à trois dimensions du référentiel du système de navigation est inférieure à une valeur seuil. Cette valeur seuil peut par exemple avoir pour valeur 1 mm, voire 0,5 mm.

Tant que les parties métalliques du bras articulé du robot médical restent dans cette région, il est possible de suivre avec précision la position des deux capteurs, et ainsi de déterminer la position de l'anatomie d'intérêt par rapport au robot médical.

De telles dispositions permettent de déterminer la position de l'anatomie d'intérêt du patient par rapport au robot médical pendant toute la durée de l'intervention, même si des parties métalliques du robot se trouvent à proximité de l'anatomie d'intérêt, et même si des objets occultent une ligne de vue entre l'anatomie d'intérêt et un dispositif de contrôle du système de navigation.

Dans des modes particuliers de mise en œuvre, l'invention peut comporter en outre l'une ou plusieurs des caractéristiques suivantes, prises isolément ou selon toutes les combinaisons techniquement possibles.

Dans des modes particuliers de mise en œuvre, le générateur de champ électromagnétique prend la forme d'une plaque, et la région d'influence réduite est déterminée comme étant une région de la zone de mesure située à l'opposé de ladite plaque par rapport à un plan parallèle à la plaque et passant par le capteur qui est à la distance la plus grande de la plaque dans une direction orthogonale à la plaque.

Dans des modes particuliers de mise en œuvre, la région d'influence réduite est déterminée comme étant une région de la zone de mesure qui est située à l'opposé du générateur de champ électromagnétique par rapport à un plan orthogonal à une direction passant par un centre du générateur de champ électromagnétique et par le capteur qui est à la plus grande distance du centre du générateur de champ électromagnétique.

De telles dispositions permettent de s'assurer que les parties métalliques du bras articulé du robot médical restent « au-delà » d'un plan tangent à une ligne de champ passant par ledit capteur. Lesdites parties métalliques ne peuvent donc pas couper les lignes de champ passant par les capteurs, et par conséquent elles ne perturbent quasiment pas les mesures effectuées par le système de navigation.

Dans des modes particuliers de mise en œuvre, le bras articulé est configuré en fonction d'un plan d'intervention mémorisé dans l'unité de contrôle du robot médical. Ledit plan d'intervention comporte des informations sur au moins une action à effectuer sur l'anatomie d'intérêt du patient. La configuration du bras articulé est déterminée à partir des positions des capteurs déterminées par le système de navigation pour permettre au praticien de réaliser ladite action ultérieurement au procédé de positionnement.

De telles dispositions permettent de suivre un déplacement de l'anatomie d'intérêt, c'est-à-dire d'ajuster la position du bras articulé du robot médical par rapport à la position de l'anatomie d'intérêt du patient, lorsque l'anatomie d'intérêt du patient subit des déplacements dus par exemple à la respiration du patient, à des manipulations intra-opératoires ou à des mouvements internes de l'anatomie d'intérêt. L'invention permet ainsi un recalage automatique de la position du bras articulé du robot médical en fonction de mouvements de l'anatomie d'intérêt du patient.

Dans des modes particuliers de mise en œuvre, un outil peut être monté sur un porte-outil monté à une extrémité libre du bras articulé, et une action du plan d'intervention correspond à permettre le placement de l'outil à une position prédéterminée, dans un volume prédéterminé ou selon une trajectoire prédéterminée, par rapport à ladite anatomie d'intérêt du patient.

On entend par « extrémité libre » l'extrémité du bras articulé qui n'est pas reliée à la base du robot médical.

Il convient de noter que le geste médical en tant que tel (par exemple l'insertion de l'aiguille dans l'anatomie d'intérêt du patient) est réalisé par le praticien ultérieurement au procédé de positionnement du bras articulé du robot médical. L'exécution d'un tel geste médical ne fait donc pas partie du procédé de positionnement du bras articulé.

Dans des modes particuliers de mise en œuvre, une configuration du bras articulé permettant d'effectuer une action du plan d'intervention est déterminée à partir :
- d'une image préopératoire comportant des informations sur ladite action planifiée à effectuer sur l'anatomie d'intérêt du patient,
- d'une image intra-opératoire comportant des informations sur la position de l'anatomie d'intérêt du patient par rapport à la position du premier capteur situé au niveau de l'anatomie d'intérêt,
- d'un recalage de l'image intra-opératoire avec l'image préopératoire pour obtenir une image comportant à la fois les informations sur l'action planifiée à effectuer sur l'anatomie d'intérêt du patient et les informations sur la position de ladite anatomie d'intérêt par rapport à la position dudit premier capteur.

Le plan d'intervention peut en effet être déterminé par le praticien pendant une phase de planification basée sur des images médicales de type scanner, tomodensitométrie (CT pour « computerized tomography » en anglais), imagerie par résonance magnétique (IRM), tomographie par émission de positons (« positron emission tomography » ou PET en anglais), ultrasons, rayons X, etc. Le praticien peut alors choisir une position ou une trajectoire de l'instrument médical par rapport à une anatomie d'intérêt du patient sur une ou plusieurs images médicales.

La position réelle de l'anatomie d'intérêt du patient au moment de l'intervention ne correspond cependant pas nécessairement à une position prévue ou modélisée pendant une phase de planification préopératoire. Il est donc avantageux de pouvoir recaler une image préopératoire à partir de laquelle une action à réaliser sur l'anatomie d'intérêt est planifiée avec une image intra-opératoire représentant précisément la position de l'anatomie d'intérêt du patient au moment de l'intervention.

La position de l'anatomie d'intérêt du patient au moment de l'intervention peut être déterminée sur des images médicales de type scanner, CT, IRM, PET, ultrasons, rayons X, fluoroscopie, tomographie volumique à faisceau conique (CBCT pour « Cone Beam computed tomography » en anglais), etc.

Dans des modes particuliers de mise en œuvre, le premier capteur comporte des éléments visibles sur ladite image intra-opératoire, et la géométrie desdits éléments est connue.

De telles dispositions permettent de déterminer avec précision la position du premier capteur par rapport à l'anatomie d'intérêt sur l'image intra-opératoire.

Dans des modes particuliers de mise en œuvre, l'anatomie d'intérêt du patient et le premier capteur électromagnétique sont situés à l'intérieur du corps du patient. Avec un système de navigation électromagnétique, il n'est en effet pas nécessaire que le capteur positionné au niveau de l'anatomie d'intérêt du patient soit visible. Selon un deuxième aspect, la présente invention concerne un robot médical pour assister un praticien lors d'une intervention médicale sur une anatomie d'intérêt d'un patient. Le robot médical comporte une base à laquelle est relié un bras articulé, ainsi qu'une unité de contrôle permettant de configurer le bras articulé dans une position déterminée. Le robot médical est destiné à être assisté par un système de navigation électromagnétique comportant un générateur de champ électromagnétique et deux capteurs électromagnétiques. Le champ électromagnétique généré par ledit générateur forme une zone de mesure dans laquelle la position d'un capteur peut être déterminée par le système de navigation électromagnétique et communiquée au robot médical. Un premier capteur est positionné au niveau de l'anatomie d'intérêt, un deuxième capteur est positionné sur le bras articulé. L'unité de contrôle est configurée, lorsque les deux capteurs se trouvent dans la zone de mesure, pour déterminer une région dite « d'influence réduite » de la zone de mesure à partir de la position du générateur de champ électromagnétique et à partir de la position des capteurs, et pour configurer le bras articulé de sorte que toute partie métallique du bras articulé se trouvant dans la zone de mesure est située dans ladite région d'influence réduite.

Dans des modes particuliers de réalisation, l'invention peut comporter en outre l'une ou plusieurs des caractéristiques suivantes, prises isolément ou selon toutes les combinaisons techniquement possibles.

Dans des modes particuliers de réalisation, le bras articulé comporte un porte-outil à une extrémité libre du bras articulé. Le porte-outil est réalisé dans un matériau amagnétique, et le deuxième capteur est positionné au niveau du porte-outil.

On entend par « matériau amagnétique » un matériau qui n'est pas (ou très peu) attiré ou repoussé par un aimant. Par exemple, le porte-outil est réalisé en plastique, en céramique, ou en inox série 300.

Dans des modes particuliers de réalisation, le porte-outil s'étend selon une direction longitudinale de la dernière section du bras articulé à l'extrémité libre du bras articulé, le porte-outil a une longueur au moins égale à 10 cm, et le deuxième capteur est positionné à une extrémité distale du porte-outil.

De telles dispositions permettent d'avoir une marge supplémentaire pour s'assurer que les parties métalliques du robot médical sont suffisamment éloignées du deuxième capteur afin de ne pas perturber, ou alors seulement de façon négligeable, la mesure de la position dudit capteur par le système de navigation. Dans des modes particuliers de réalisation, le générateur de champ électromagnétique prend la forme d'une plaque, et la région d'influence réduite est déterminée par l'unité de contrôle comme étant une région de la zone de mesure située à l'opposé de ladite plaque par rapport à un plan parallèle à la plaque et passant par le capteur qui est à la distance la plus grande de la plaque dans une direction orthogonale à la plaque.

Dans des modes particuliers de réalisation, la région d'influence réduite est déterminée par l'unité de contrôle comme étant une région de la zone de mesure qui est située à l'opposé du générateur de champ électromagnétique par rapport à un plan orthogonal à une direction passant par un centre du générateur de champ électromagnétique et par le capteur qui est à la plus grande distance du centre du générateur de champ électromagnétique.

Dans des modes particuliers de réalisation, le bras articulé est configuré par l'unité de contrôle en fonction d'un plan d'intervention mémorisé dans l'unité de contrôle du robot médical. Ledit plan d'intervention comporte des informations sur au moins une action à effectuer sur l'anatomie d'intérêt du patient. La configuration du bras articulé est déterminée à partir des positions des capteurs déterminées par le système de navigation pour permettre au praticien de réaliser ladite action ultérieurement au procédé de positionnement.

Dans des modes particuliers de réalisation, une configuration du bras articulé permettant d'effectuer une action du plan d'intervention est déterminée à partir :
- d'une image préopératoire comportant des informations sur ladite action planifiée à effectuer sur l'anatomie d'intérêt du patient,
- d'une image intra-opératoire comportant des informations sur la position de l'anatomie d'intérêt du patient par rapport à la position du premier capteur situé au niveau de l'anatomie d'intérêt,
- d'un recalage de l'image intra-opératoire avec l'image préopératoire pour obtenir une image comportant à la fois les informations sur l'action planifiée à effectuer sur l'anatomie d'intérêt du patient et les informations sur la position de ladite anatomie d'intérêt par rapport à la position dudit premier capteur.

### Présentation des figures

L'invention sera mieux comprise à la lecture de la description suivante, donnée à titre d'exemple nullement limitatif, et faite en se référant aux figures 1 à 7 qui représentent :
[Fig.1] une représentation schématique d'un robot médical selon l'invention,
[Fig.2] une représentation schématique, vue de dessus, du placement d'un générateur de champ d'un système de navigation électromagnétique et de la zone de mesure correspondante pour un patient allongé sur une table d'intervention,
[Fig.3] une représentation schématique d'une région dite « d'influence réduite » de la zone de mesure,
[Fig.4] une représentation schématique de la détermination d'une région dite « d'influence réduite » de la zone de mesure pour un mode particulier de mise en œuvre de l'invention,
[Fig.5] une représentation schématique de la détermination d'une région dite « d'influence réduite » de la zone de mesure pour un mode particulier de mise en œuvre de l'invention,
[Fig.6] une autre représentation schématique du mode particulier de mise en œuvre de l'invention décrit en référence à la figure 5,
[Fig.7] une représentation schématique des principales étapes d'un procédé de positionnement d'un bras articulé d'un robot médical selon l'invention.

Dans ces figures, des références identiques d'une figure à une autre désignent des éléments identiques ou analogues. Pour des raisons de clarté, les éléments représentés ne sont pas nécessairement à une même échelle, sauf mention contraire.

### Description détaillée d'un mode de réalisation de l'invention

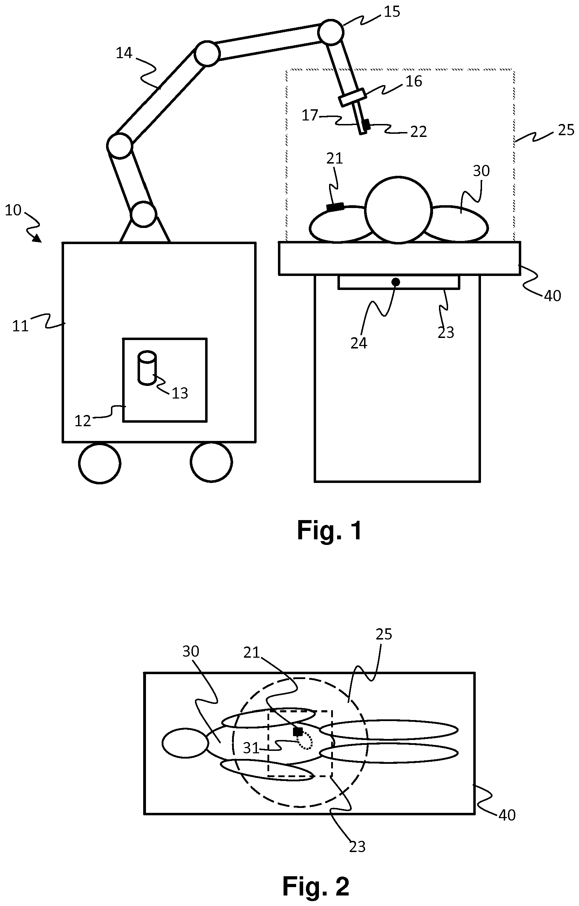

La figure 1 représente schématiquement un robot médical 10 selon l'invention ainsi qu'un patient 30 allongé sur une table d'intervention.

Le robot médical 10 comporte une base 11. Dans l'exemple illustré à la figure 1, la base 11 du robot médical 10 est équipée de roues, ce qui permet au robot médical 10 de se déplacer selon différentes directions par des mouvements de translation et/ou de rotation.

Le robot médical 10 comporte un bras 14 articulé dont une extrémité est reliée à la base 11. Un outil médical peut être monté sur un porte-outil 17 à l'extrémité libre du bras 14 articulé, c'est-à-dire à l'extrémité du bras 14 articulé qui n'est pas reliée à la base 11 du robot médical 10.

Un tel robot médical 10 peut alors être utilisé pour aider un praticien à positionner, maintenir, ou guider l'outil médical. Le robot médical 10 joue alors le rôle d'une troisième main pour le praticien.

Le bras 14 articulé comporte de préférence au moins six degrés de liberté pour pouvoir positionner et/ou déplacer l'outil médical dans un espace à trois dimensions. De manière encore plus préférentielle, le bras articulé comporte 7 degrés de liberté permettant de changer la configuration du bras en conservant la position de l'outil. Par exemple, l'outil peut être un guide pour implanter un instrument tel qu'une aiguille, un cathéter, ou une électrode dans une zone cible d'une anatomie d'intérêt du patient 30.

Selon un autre exemple, l'outil peut être un instrument médical, une sonde ou une électrode qu'il convient d'introduire très précisément dans un organe cible pour permettre une biopsie, une résection ou une ablation d'une partie de l'anatomie d'intérêt du patient 30.

Dans la suite de la description, on se place à titre d'exemple et de manière non limitative dans le cas où un porte-outil 17 est monté à l'extrémité libre du bras 14 articulé du robot médical 10, et l'outil monté sur le porte-outil 17 est un instrument de guidage pour guider une aiguille. Tel qu'illustré sur la figure 1, le porte-outil 17 est par exemple monté sur le bras 14 articulé du robot médical 10 par l'intermédiaire d'une bride 16.

Il est important que le robot médical 10 puisse positionner l'outil à une position prédéterminée, ou déplacer l'outil dans un espace prédéterminé ou selon une trajectoire prédéterminée, avec précision par rapport à l'anatomie d'intérêt du patient 30.

Le bras 14 articulé comporte une ou plusieurs articulations 15 contrôlées par une unité de contrôle 12 du robot médical 10. Une configuration possible du bras 14 articulé correspond alors à un ensemble de valeurs de paramètres prises par la ou les articulations 15 du bras 14 articulé (par exemple un angle de rotation, une distance de translation, etc.).

L'unité de contrôle 12 comporte par exemple un ou plusieurs processeurs et une mémoire 13 (disque dur magnétique, mémoire électronique, disque optique, etc.) dans laquelle est mémorisée un produit programme d'ordinateur, sous la forme d'un ensemble d'instructions de code de programme à exécuter pour mettre en œuvre les différentes étapes d'un procédé de positionnement du bras 14 articulé du robot médical 10. Alternativement ou en complément, l'unité de contrôle 12 comporte un ou des circuits logiques programmables (FPGA, PLD, etc.), et/ou un ou des circuits intégrés spécialisés (ASIC), et/ou un ensemble de composants électroniques discrets, etc., adaptés à mettre en œuvre tout ou partie desdites étapes dudit procédé de positionnement.

Un système de navigation électromagnétique est utilisé pour permettre de déterminer la position de l'anatomie d'intérêt du patient 30 par rapport au robot médical 10. Le système de navigation électromagnétique comporte un générateur 23 de champ électromagnétique ainsi que deux capteurs 21, 22 électromagnétiques. Un premier capteur 21 est placé au niveau de l'anatomie d'intérêt 31 du patient 30. Un deuxième capteur 22 est monté sur le bras 14 articulé du robot médical 10, à une position connue dans un repère du robot médical 10.

Le système de navigation peut également comporter un dispositif de contrôle centralisé configuré pour déterminer la position des capteurs 21, 22 à partir de mesures réalisées par lesdits capteurs 21, 22. Un tel dispositif de contrôle n'est pas représenté sur les figures. Il peut éventuellement être intégré avec le générateur 23 de champ électromagnétique. Le dispositif de contrôle est configuré pour communiquer, par exemple via des moyens de communication sans fil, la position des capteurs 21, 22 à l'unité de contrôle 12 du robot médical 10.

De manière connue, les capteurs 21, 22 électromagnétiques comprennent par exemple au moins deux bobines conductrices qui peuvent être configurées pour mesurer six degrés de liberté lorsque lesdits capteurs 21, 22 sont soumis à un champ électromagnétique externe. Chaque bobine d'un capteur 21, 22 électromagnétique produit un signal électrique induit ayant des caractéristiques qui dépendent de la position de la bobine par rapport au champ électromagnétique.

Le système de navigation permet ainsi de déterminer la position du premier capteur 21 et la position du deuxième capteur 22 dans un repère du système de navigation lorsque les capteurs 21, 22 se trouvent dans une zone de mesure 25 du système de navigation. Un tel repère est par exemple centré par rapport à un centre 24 du générateur 23 de champ électromagnétique.

La zone de mesure 25 correspond à une région de l'espace dans laquelle le champ électromagnétique émis par le générateur 23 est suffisamment fort pour permettre de déterminer la position d'un capteur 21, 22 électromagnétique se trouvant dans cette zone. Dans l'exemple considéré, et tel qu'illustré aux figures 1 et 2, le générateur 23 de champ électromagnétique du système de navigation est une plaque fixée horizontalement sous une table 40 d'intervention sur laquelle le patient 30 est allongé. La zone de mesure 25 prend la forme d'un cylindre d'un diamètre d'environ 500 mm et d'une hauteur d'environ 600 mm s'étendant au-dessus de la table 40 d'intervention et dont une base est centrée sur la plaque formée par le générateur 23. Tel qu'illustré sur la figure 2, le générateur 23 de champ électromagnétique est fixée sous la table 40 d'intervention à une position telle qu'un capteur 21 placé au niveau de l'anatomie d'intérêt 31 du patient 30 est inclus dans la zone de mesure 25.

Le système de navigation électromagnétique permet de déterminer la position du premier capteur 21 et la position du deuxième capteur 22 dans un repère du système de navigation électromagnétique. La position du premier capteur 21 et la position du deuxième capteur 22 sont donc connues l'une par rapport à l'autre. Comme la position du deuxième capteur 22 est connue dans un repère du robot médical 10, la position du premier capteur 21 peut être déterminée dans un repère du robot médical 10.

La position de l'anatomie d'intérêt 31 du patient 30 est connue par rapport à la position du premier capteur 21. Par exemple, une image intra-opératoire sur laquelle apparaît à la fois l'anatomie d'intérêt 31 du patient 30 et la position du premier capteur 21 peut être réalisée pendant l'intervention médicale.

Pour apparaître sur une image intra-opératoire, le premier capteur 21 peut par exemple comporter des billes céramiques radio-opaques. La géométrie desdites billes est connue afin de pouvoir déterminer avec précision la position du premier capteur 21 par rapport à l'anatomie d'intérêt 31 sur l'image intra-opératoire. Connaissant la position de l'anatomie d'intérêt 31 par rapport au premier capteur 21 et la position du premier capteur 21 par rapport au robot médical 10, il est possible de déterminer la position de l'anatomie d'intérêt 31 par rapport robot médical 10. Ainsi, tout au long de l'intervention médicale, la configuration du bras 14 articulé du robot médical 10 peut être déterminée en fonction de la position des capteurs 21, 22 afin de placer et maintenir un outil médical à une position prédéterminée, déplacer l'outil dans un espace prédéterminé et/ou selon une trajectoire prédéterminée, avec précision, par rapport à l'anatomie d'intérêt 31 du patient 30.

En particulier, il est possible de suivre des mouvements de l'anatomie d'intérêt 31 et d'ajuster, ou autrement dit de recaler, la position du bras 14 articulé du robot médical 10 en fonction de ces mouvements. Des mouvements de l'anatomie d'intérêt 31 du patient 30 peuvent être dus par exemple à la respiration du patient, à des manipulations intra-opératoire sur le patient 30, ou à des mouvements d'organes internes du patient 30.

Dans des modes particuliers de mise en œuvre, le bras 14 articulé est configuré en fonction d'un plan d'intervention mémorisé dans l'unité de contrôle 12 du robot médical 10.

Le plan d'intervention comporte des informations sur une ou plusieurs actions à réaliser sur l'anatomie d'intérêt 31 du patient 30. Une action du plan d'intervention correspond par exemple à pouvoir manipuler un outil médical dans un espace prédéterminé, à placer l'outil dans une position prédéterminée, ou bien à déplacer l'outil selon une trajectoire prédéterminée par rapport à l'anatomie d'intérêt 31 du patient 30.

Le plan d'intervention est créé pendant une phase de planification. Cette phase de planification peut précéder l'intervention médicale, elle est alors définie à partir d'images médicales préopératoires. Selon un autre exemple, la phase de planification peut être définie pendant l'intervention à partir d'images médicales intra-opératoires.

Lors de cette phase de planification, le praticien définit les différentes actions qui devront être effectuées sur l'anatomie d'intérêt. Le plan d'intervention peut par exemple être généré à l'aide d'images médicales de type CT scan, IRM, PET, ultrasons, rayons X ou autre. Un opérateur, généralement le praticien qui effectuera l'intervention à l'aide du robot médical 10, sélectionne les paramètres de l'outil (par exemple une longueur, un diamètre, une forme 3D, une puissance d'énergie à délivrer, une intensité de courant, un temps de traitement, etc.). Une ou plusieurs actions peuvent être planifiées selon le type de traitement à effectuer. La planification peut être complètement manuelle, interactive, ou complètement automatisée à l'aide d'algorithmes de segmentation et de planification. Ces algorithmes d'aide à la décision peuvent être par exemple basés sur des systèmes experts (système capable de reproduire les mécanismes cognitifs du praticien en effectuant un raisonnement à partir de faits et de règles connues) ou sur des mécanismes intelligents d'apprentissage automatique (par exemple avec des réseaux de neurones convolutifs).

Les actions du plan d'intervention sont par exemple encodées sous la forme d'instructions connues par l'unité de contrôle 12 dans un fichier informatique. Le fichier informatique correspondant au plan d'intervention peut, par exemple, être généré sur un ordinateur distinct du robot médical 10. Le fichier est alors transmis au robot médical 10 et mémorisé dans la mémoire 13 de l'unité de contrôle 12. Cette transmission du fichier informatique peut être réalisée sous différentes formes, de manière conventionnelle, comme par exemple par une transmission de fichier par clé USB (acronyme anglais de « Universal Serial Bus »), ou bien par une communication sans fil.

Les actions du plan d'intervention décrivent par exemple les différentes positions ou les différents mouvements de l'outil par rapport à l'anatomie d'intérêt 31 du patient 30. L'unité de contrôle 12 connaît en outre le modèle géométrique du bras 14 articulé et de l'outil. Par exemple, le bras 14 est équipé de codeurs qui permettent de connaître la position angulaire de chacun de ses axes et, par calcul, de connaître la position de l'outil. L'unité de contrôle 12 peut alors déterminer à partir des informations contenues dans le plan d'intervention une ou plusieurs configurations du bras 14 articulé pour lesquelles le praticien sera capable de réaliser les actions prévues par le plan d'intervention.

Le geste médical en tant que tel, par exemple l'insertion de l'aiguille dans l'anatomie d'intérêt 31 du patient 30, n'est exécuté par le praticien qu'ultérieurement au procédé de positionnement du bras 14 articulé du robot médical 10. L'exécution d'un tel geste médical ne fait donc pas partie du procédé de positionnement du bras 14 articulé selon l'invention.

Il est avantageux pour le praticien de pouvoir définir le plan d'intervention dans une phase de planification qui précède l'intervention médicale, par exemple plusieurs jours ou plusieurs heures avant l'intervention. La position réelle de l'anatomie d'intérêt 31 du patient 30 au moment de l'intervention ne correspond cependant pas nécessairement à une position prévue ou modélisée pendant une phase de planification préopératoire. Il est donc avantageux de pouvoir recaler une image préopératoire à partir de laquelle une action à réaliser sur l'anatomie d'intérêt 31 est planifiée avec une image intra-opératoire représentant précisément la position de l'anatomie d'intérêt 31 du patient 30 au moment de l'intervention.

Ainsi, dans des modes particuliers de mise en œuvre, une configuration du bras 14 articulé permettant d'effectuer une action du plan d'intervention est déterminée à partir :
- d'une image préopératoire comportant des informations sur ladite action,
- d'une image intra-opératoire comportant des informations sur la position de l'anatomie d'intérêt 31 du patient 30 par rapport à la position du premier capteur 21 situé au niveau de l'anatomie d'intérêt 31,

- d'un recalage de l'image intra-opératoire avec l'image préopératoire pour obtenir une image comportant à la fois les informations sur l'action planifiée à effectuer sur l'anatomie d'intérêt du patient 30 et les informations sur la position de ladite anatomie d'intérêt 31 par rapport à la position dudit capteur 21. Il existe différentes méthodes de recalage d'une image avec une autre. De telles méthodes sont considérées comme connues de l'homme du métier.

Un inconvénient du système de navigation électromagnétique est que la précision de la mesure d'une position d'un capteur 21, 22 par le système de navigation peut être perturbée par la présence d'un objet métallique dans la zone de mesure 25. Notamment, la bride 16 sur laquelle est montée le porte-outil 17 et les articulations 15 motorisées du bras 14 articulé du robot médical 10 comportent généralement des pièces métalliques. Si une pièce métallique intercepte une ligne de champ 27 du champ électromagnétique émis par le générateur 23 passant par un capteur 21, 22, elle causera une distorsion du champ électromagnétique perçu par ledit capteur 21, 22. La détermination de la position dudit capteur 21, 22 par le système de navigation sera alors faussée.

Il convient alors de s'assurer que les parties métalliques du bras 14 articulé du robot médical 10 se trouvant dans la zone de mesure 25 ne perturbent pas, ou alors seulement de façon négligeable, les mesures des positions des capteurs 21, 22 par le système de navigation.

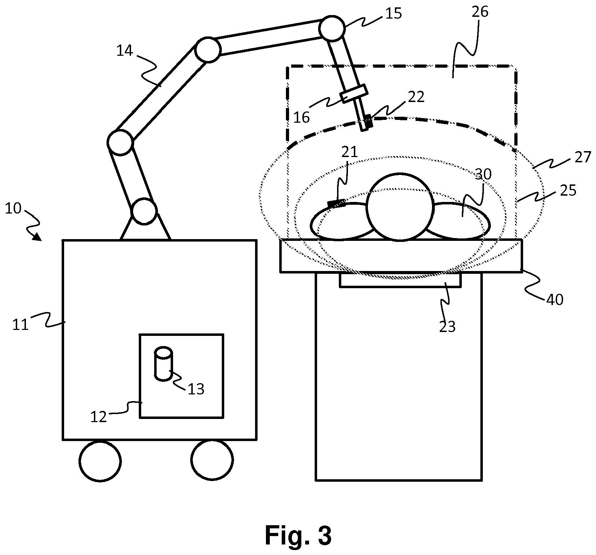

Dans ce but, et tel qu'illustré schématiquement sur la figure 3, l'unité de contrôle 12 est configurée pour déterminer une région dite « d' nfluence réduite » 26 de la zone de mesure 25 dans laquelle l'introduction d'un objet métallique ne perturbe quasiment pas la détermination de la position du premier capteur 21 et la position du deuxième capteur 22 par le système de navigation électromagnétique lorsque lesdits capteurs 21, 22 se trouvent dans la zone de mesure du champ électromagnétique généré par le générateur 23 de champ électromagnétique du système de navigation électromagnétique. Idéalement, la région d'influence réduite 26 est définie de telle sorte que toute partie métallique du bras 14 articulé du robot médical 10 se trouvant dans la zone de mesure 25 ne coupe pas une ligne de champ 27 passant par un capteur 21, 22.

L'unité de contrôle 12 du robot médical 10 est également adaptée pour configurer le bras 14 articulé du robot médical 10 de telle sorte que, pendant l'intervention médicale, toute partie métallique du bras 14 articulé se trouvant dans la zone de mesure 25 est située à l'intérieur de la région d'influence réduite 26.

Pour un bras 14 articulé du robot médical 10 ayant plus de degrés de liberté que nécessaire, plusieurs configurations du bras 14 articulé permettant d'effectuer une action planifiée peuvent être envisagées. Par exemple, un bras 14 articulé anthropomorphe à six degrés de liberté peut généralement positionner un guide-aiguille selon une direction rectiligne passant par un point d'entrée prédéterminé selon plusieurs configurations différentes de ses axes. L'unité de contrôle 12 du robot médical 10 peut alors généralement choisir une configuration du bras 14 articulé qui est compatible avec la contrainte selon laquelle toute partie métallique du bras 14 articulé se trouvant dans la zone de mesure 25 doit être située à l'intérieur de la région d'influence réduite 26.

La région d'influence réduite est recalculée au fur et à mesure que la position des capteurs 21, 22 changent, afin de s'assurer que cette contrainte est toujours vérifiée au cours de l'intervention.

Tant que les parties métalliques du bras 14 articulé du robot médical 10 restent dans cette région d'influence réduite 26, il est possible de suivre avec précision la position des deux capteurs 21, 22, et donc de déterminer avec précision la position de l'anatomie d'intérêt 31 par rapport au robot médical 10.

De telles dispositions permettent de déterminer la position de l'anatomie d'intérêt 31 du patient 30 par rapport au robot médical 10 pendant toute la durée de l'intervention, même si des parties métalliques du robot se trouvent relativement proches de l'anatomie d'intérêt 31, et même si l'anatomie d'intérêt 31 du patient 30 subit des mouvements au cours de l'intervention médicale.

Il est alors possible de recaler automatiquement la position du bras 14 articulé du robot médical 10 en fonction des mouvements de l'anatomie d'intérêt 31 pendant l'intervention médicale.

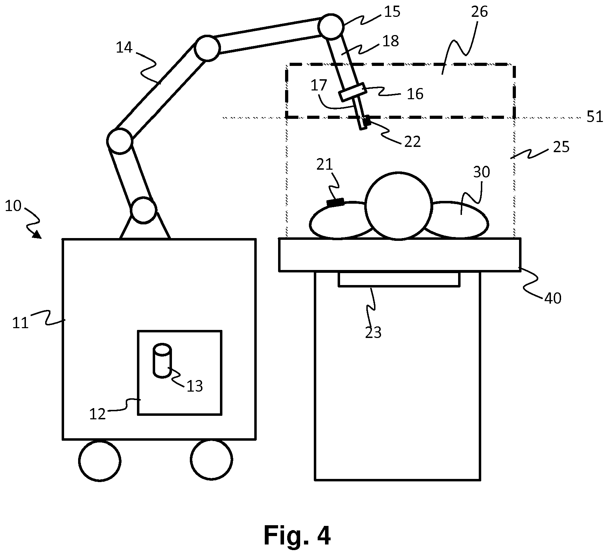

La figure 4 représente schématiquement un mode particulier de mise en œuvre pour la détermination de la région d'influence réduite 26. Dans ce mode particulier de mise en œuvre, le générateur 23 de champ électromagnétique prend la forme d'une plaque, et la région d'influence réduite 26 est la région de la zone de mesure 25 située à l'opposé de ladite plaque par rapport à un plan 51 parallèle à la plaque et passant par le capteur 21, 22 qui est à la distance la plus grande de la plaque dans une direction orthogonale à la plaque.

Dans l'exemple illustré à la figure 4, le deuxième capteur 22 situé sur le bras 14 articulé du robot médical 10 est le capteur le plus éloigné du générateur 23 dans une direction orthogonale à la plaque formée par le générateur 23. La partie supérieure de la zone de mesure 25 délimitée par le plan 51 correspond donc à la région d'influence réduite 26 dans laquelle des parties métalliques du bras 14 articulé peuvent être introduites sans perturber significativement la détermination de la position des capteurs 21, 22 par le système de navigation.

En déterminant ainsi la région d'influence réduite 26, les parties métalliques du bras 14 articulé restent en permanence « au-dessus » des capteurs 21, 22 par rapport au générateur 23 de champ électromagnétique.

La détermination de la région d'influence réduite 26 selon le mode particulier de mise en œuvre décrit en référence à la figure 4 est simple et particulièrement bien adaptée lorsque la dernière section 18 du côté de l'extrémité libre du bras 14 articulé doit agir sur l'anatomie d'intérêt 31 du patient 30 selon une direction sensiblement verticale par rapport à table 40 et par rapport au générateur 23. Elle n'est cependant pas bien adaptée aux cas où la dernière section 18 du bras 14 articulé doit agir sur l'anatomie d'intérêt 31 du patient 30 selon une direction sensiblement horizontale par rapport à table 40 et par rapport au générateur 23.

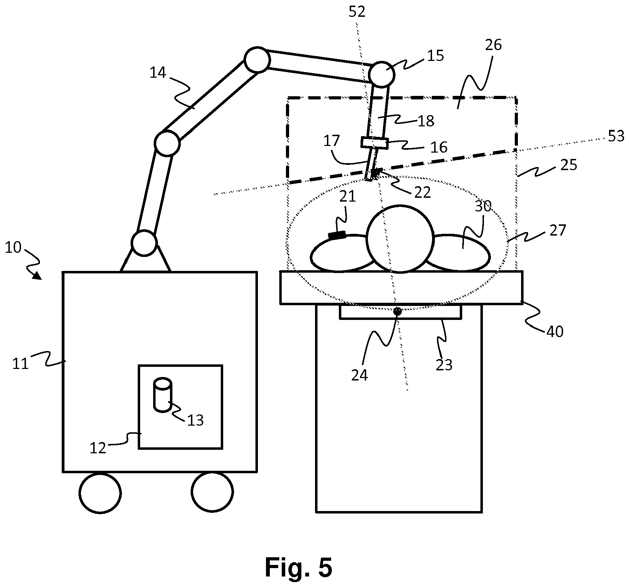

La figure 5 représente schématiquement un autre mode particulier de mise en œuvre pour la détermination de la région d'influence réduite 26. Dans ce mode particulier de mise en œuvre, la région d'influence réduite 26 est une région de la zone de mesure 25 qui est située à l'opposé du générateur 23 de champ électromagnétique par rapport à un plan 53 orthogonal à une direction 52 passant par un centre 24 du générateur 23 de champ électromagnétique et par le capteur 21, 22 qui est à la plus grande distance du centre 24 du générateur 23 de champ électromagnétique.

Il convient de noter que la position du centre 24 du générateur 23 de champ électromagnétique et la position de la zone de mesure 25 peuvent être déterminées par l'unité de contrôle 12 dans un repère du robot médical 10 car d'une part la position du deuxième capteur 22 est connue dans un repère du robot médical 10 et d'autre part la position du générateur 23 peut être déterminée par rapport à la position dudit deuxième capteur 22 dans un repère du système de navigation. Des matrices de transformation peuvent alors permettre, de manière conventionnelle, de déterminer la position du générateur 23 dans un repère du robot médical 10. La zone de mesure 25 est quant à elle connue, car spécifiée pour le système de navigation utilisé, par rapport à la position du générateur 23 de champ électromagnétique. Les spécifications de la zone de mesure 25 peuvent par exemple être sauvegardées dans la mémoire 13 de l'unité de contrôle 12 du robot médical 10. Dans l'exemple illustré sur la figure 5, le deuxième capteur 22 situé sur le bras 14 articulé du robot médical 10 est le capteur le plus éloigné du centre 24 du générateur 23. Le plan 53 est un plan sensiblement tangent à une ligne de champ 27 passant par le capteur 22. Tant que les parties métalliques du bras 14 articulé du robot médical 10 restent « au-delà » de ce plan 53 par rapport au générateur 23, elles ne peuvent pas couper les lignes de champ passant par les capteurs 21, 22, et par conséquent elles ne perturbent que de façon négligeable les mesures effectuées par le système de navigation.

Il convient de noter que dans ce mode particulier de réalisation, il importe peu que le générateur 23 ait une forme de plaque ou bien qu'il ait une autre forme.

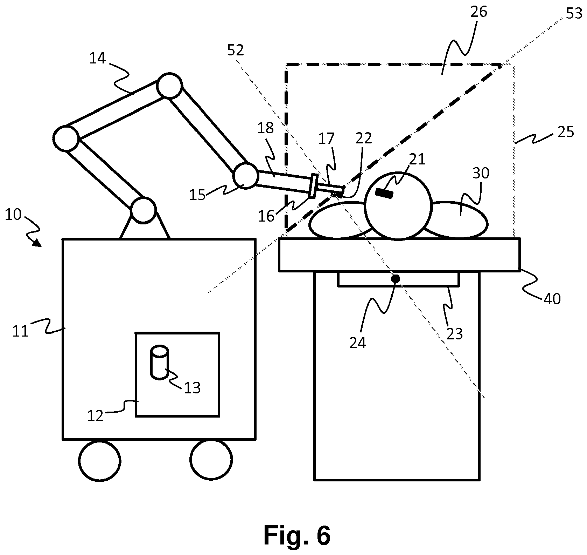

La figure 6 représente une région d'influence réduite 26 déterminée avec le même mode de mise en œuvre que celui décrit en référence à la figure 5 lorsque la dernière section 18 du côté de l'extrémité libre du bras 14 articulé du robot médical 10 prend une position sensiblement horizontale.

Il apparaît bien que cette méthode de détermination de la région d'influence réduite 26 n'empêche pas une telle configuration du bras 14 articulé du robot médical 10.

Dans des modes particuliers de réalisation, le bras 14 articulé comporte un porte-outil 17 à une extrémité libre du bras 14 articulé. Le porte-outil est réalisé dans un matériau amagnétique, c'est-à-dire un matériau qui n'est pas (ou peu) attiré ou repoussé par un aimant. A titre d'exemples nullement limitatifs, le porte-outil peut par exemple être réalisé en plastique, en céramique ou en inox série 300. Le deuxième capteur 22, positionné sur le bras 14 articulé, est positionné au niveau du porte-outil 17.

De préférence, le porte-outil 17 s'étend selon une direction longitudinale de la dernière section du bras 14 articulé à l'extrémité libre du bras 14 articulé, le porte-outil 17 a une longueur au moins égale à 10 cm, et le deuxième capteur 22 est positionné à une extrémité distale du porte-outil 17.

De telles dispositions permettent d'avoir une marge supplémentaire pour s'assurer que les parties métalliques du robot médical 10 sont suffisamment éloignées du deuxième capteur 22 afin de ne pas perturber significativement la mesure de la position dudit deuxième capteur 22 par le système de navigation. En fonction des matériaux du robot médical 10 et du type du générateur 23 de champ électromagnétique utilisés, la longueur du porte-outil 17 pourrait être différente. Notamment, il peut être avantageux dans certains cas d'avoir un porte-outil 17 de longueur au moins égale à 19 cm.

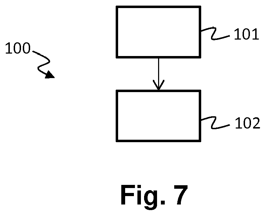

La figure 7 représente schématiquement les principales étapes d'un procédé 100 de positionnement d'un bras 14 articulé d'un robot médical 10 tel que décrit précédemment.

Avant l'exécution du procédé 100 de positionnement, on considère que le premier capteur 21 est positionné au niveau de l'anatomie d'intérêt 31 du patient 30 à traiter. On considère aussi que le patient et le générateur 23 de champ électromagnétique sont installés l'un par rapport à l'autre de sorte que ledit premier capteur 21 est situé dans la zone de mesure 25 du système de navigation électromagnétique. Enfin, on considère que le robot médical 10 est placé à proximité du patient 30, à une position permettant au bras 14 articulé du robot médical 10 de réaliser l'ensemble des actions à effectuer sur l'anatomie d'intérêt 31.

Il existe différentes méthodes pour placer le robot médical 10 à une position à laquelle le bras 14 articulé du robot médical 10 est capable de réaliser l'ensemble des actions à effectuer sur l'anatomie d'intérêt 31. Il peut s'agir d'une méthode complètement manuelle, dans laquelle un opérateur déplace le robot médical 10 à une telle position. Il peut aussi s'agir d'une méthode complètement automatisée, dans laquelle le robot médical 10 comporte par exemple des moyens de repérage dans l'espace (caméras, télémètres, capteurs inertiels, optiques ou odométriques, etc.) et des moyens de calculs pour détecter une telle position et s'y positionner de manière autonome.

A partir de là, le procédé 100 de positionnement automatique du bras 14 articulé du robot médical 10 selon l'invention peut être mis en œuvre pour détecter et suivre avec précision la position de l'anatomie d'intérêt 31 au cours de l'intervention et configurer le bras 14 articulé dans des positions qui conviennent à la fois pour permettre au praticien de réaliser les actions à effectuer sur l'anatomie d'intérêt et pour ne quasiment pas perturber les mesures du système de navigation électromagnétique permettant de déterminer la position des capteurs 21, 22.

Le procédé 100 de positionnement du bras 14 articulé comporte notamment les étapes suivantes :
- une détermination 101, lorsque les deux capteurs 21, 22 se trouvent dans la zone de mesure 25, d'une région dite « d'influence réduite » 26 de la zone de mesure 25 à partir de la position du générateur 23 de champ électromagnétique et à partir de la position des capteurs 21, 22,
- une configuration 102 du bras 14 articulé de telle sorte que toute partie métallique du bras 14 articulé se trouvant dans la zone de mesure 25 est située à l'intérieur de la région d'influence réduite 26.

La description ci-avant illustre clairement que, par ses différentes caractéristiques et leurs avantages, la présente invention atteint les objectifs fixés.

En particulier, le robot médical 10 peut intervenir dans n'importe quelle salle d'intervention, il n'est pas lié à une salle d'intervention particulière, et sa position n'a pas à être connue par rapport à un dispositif d'imagerie particulier. Cela limite alors les coûts relatifs aux interventions médicales assistées par un robot car il n'y a pas besoin d'associer un robot médical à chaque salle d'intervention ou bien de préparer spécifiquement une salle d'intervention pour qu'un robot médical y soit fonctionnel. Un système de navigation électromagnétique est avantageux par rapport à un système de navigation optique car il n'est pas sensible à d'éventuels obstacles occultant une ligne de vue directe entre un capteur et un dispositif de contrôle du système de navigation.

Aussi, un système de navigation électromagnétique permet de déterminer la position d'un capteur 21 placé à l'intérieur du corps humain d'un patient 30, ce qui permet de traiter des anatomies d'intérêt 31 localisées à l'intérieur du corps du patient 30. Grâce au système de navigation électromagnétique, le robot médical 10 est capable de suivre en permanence la position de l'anatomie d'intérêt 31 du patient pendant l'intervention. Le robot médical 10 peut donc ajuster la position de son bras 14 articulé en fonction des mouvements de l'anatomie d'intérêt 31 pendant l'intervention.

La détermination d'une région d'influence réduite 26 en fonction de la position des capteurs 21, 22 tout au long de l'intervention permet à l'unité de contrôle 12 du robot médical 10 de choisir des configurations du bras 14 articulé qui permettent au praticien d'exécuter les actions attendues tout en s'assurant que les mesures fournies par le système de navigation électromagnétique ne sont que faiblement perturbées par la présence de pièces métalliques dans la zone de mesure 25 du système de navigation.

De manière plus générale, il est à noter que les modes de mise en œuvre et de réalisation considérés ci-dessus ont été décrits à titre d'exemples non limitatifs, et que d'autres variantes sont par conséquent envisageables.

Notamment, différentes méthodes ont été présentées pour déterminer une région d'influence réduite 26 de la zone de mesure 25. D'autres méthodes sont cependant envisageables. De telles méthodes ne correspondent qu'à des variantes de l'invention.

## Revendications

1. Procédé (100) de positionnement d'un bras (14) articulé d'un robot médical (10) pour assister un praticien lors d'une intervention médicale sur une anatomie d'intérêt (31) d'un patient (30), le robot médical (10) comportant une base (11) à laquelle est relié le bras (14) articulé, ainsi qu'une unité de contrôle (12) permettant de configurer le bras (14) articulé dans une position déterminée, le robot médical (10) étant assisté par un système de navigation électromagnétique comportant un générateur (23) de champ électromagnétique et deux capteurs (21, 22) électromagnétiques, le champ électromagnétique généré formant une zone de mesure (25) dans laquelle la position d'un capteur (21, 22) peut être déterminée par le système de navigation électromagnétique et communiquée au robot médical (10), un premier capteur (21) étant positionné, dans une étape préalable audit procédé (100), au niveau de l'anatomie d'intérêt (31), un deuxième capteur (22) étant positionné sur le bras (14) articulé, le procédé de positionnement du bras (14) articulé étant **caractérisé en ce que** ledit procédé comporte :
- une détermination (101), lorsque les deux capteurs (21, 22) se trouvent dans la zone de mesure (25), d'une région dite « d'influence réduite » (26) de la zone de mesure (25) à partir de la position du générateur (23) de champ électromagnétique et à partir de la position des capteurs (21, 22), ladite région d'influence réduite (26) étant une région pour laquelle la présence d'une partie métallique du bras (14) articulé dans cette région perturbe de façon négligeable la détermination de la position de chacun des deux capteurs (21, 22),
- une configuration (102) du bras (14) articulé de telle sorte que toute partie métallique du bras (14) articulé se trouvant dans la zone de mesure (25) est située à l'intérieur de la région d'influence réduite (26),
ledit procédé (100) de positionnement du bras (14) articulé ne comportant pas d'étape d'exécution d'un geste médical sur le patient.

2. Procédé (100) selon la revendication 1 dans lequel le générateur (23) de champ électromagnétique prend la forme d'une plaque, et la région d'influence réduite (26) est déterminée comme étant une région de la zone de mesure (25) située à l'opposé de ladite plaque par rapport à un plan (52) parallèle à la plaque et passant par le capteur (21, 22) qui est à la distance la plus grande de la plaque dans une direction orthogonale à la plaque.

3. Procédé (100) selon la revendication 1 dans lequel la région d'influence réduite (26) est déterminée comme étant une région de la zone de mesure (25) qui est située à l'opposé du générateur (23) de champ électromagnétique par rapport à un plan (53) orthogonal à une direction (52) passant par un centre (24) du générateur (23) de champ électromagnétique et par le capteur (21, 22) qui est à la plus grande distance du centre (24) du générateur (23) de champ électromagnétique.

4. Procédé (100) selon l'une des revendications 1 à 3 dans lequel le bras (14) articulé est configuré en fonction d'un plan d'intervention mémorisé dans l'unité de contrôle (12) du robot médical (10), ledit plan d'intervention comportant des informations sur au moins une action à effectuer sur l'anatomie d'intérêt (31) du patient (30), la configuration du bras articulé étant déterminée à partir des positions des capteurs (21, 22) déterminées par le système de navigation pour permettre au praticien de réaliser ladite action ultérieurement au procédé (100) de positionnement du bras (14) articulé.

5. Procédé (100) selon la revendication 4 dans lequel une configuration du bras (14) articulé permettant d'effectuer une action du plan d'intervention est déterminée à partir :
- d'une image préopératoire comportant des informations sur ladite action,
- d'une image intra-opératoire comportant des informations sur la position de l'anatomie d'intérêt (31) du patient (30) par rapport à la position du premier capteur (21) situé au niveau de l'anatomie d'intérêt (31),
- d'un recalage de l'image intra-opératoire avec l'image préopératoire pour obtenir une image comportant à la fois les informations sur l'action planifiée à effectuer sur l'anatomie d'intérêt du patient (30) et les informations sur la position de ladite anatomie d'intérêt (31) par rapport à la position dudit premier capteur (21).

6. Procédé (100) selon la revendication 5 dans lequel le premier capteur (21) comporte des éléments visibles sur ladite image intra-opératoire, et la géométrie desdits éléments est connue.

7. Procédé (100) selon l'une des revendications 1 à 6 dans lequel l'anatomie d'intérêt (31) du patient (30) et le premier capteur (21) électromagnétique sont situés à l'intérieur du corps du patient (30).

8. Robot médical (10) pour assister un praticien lors d'une intervention médicale sur une anatomie d'intérêt (31) d'un patient (30), le robot médical (10) comportant une base (11) à laquelle est relié un bras (14) articulé, ainsi qu'une unité de contrôle (12) permettant de configurer le bras (14) articulé dans une position déterminée, le robot médical (10) étant destiné à être assisté par un système de navigation électromagnétique comportant un générateur (23) de champ électromagnétique et deux capteurs (21, 22) électromagnétiques, le champ électromagnétique généré formant une zone de mesure (25) dans laquelle la position d'un capteur (21, 22) peut être déterminée par le système de navigation électromagnétique et communiquée au robot médical (10), un premier capteur (21) étant positionné au niveau de l'anatomie d'intérêt (31), un deuxième capteur (22) étant positionné sur le bras (14) articulé, le robot médical (10) étant **caractérisé en ce que** l'unité de contrôle (12) est configurée, lorsque les deux capteurs (21, 22) se trouvent dans la zone de mesure (25), pour déterminer une région dite « d'influence réduite » (26) de la zone de mesure (25) à partir de la position du générateur (23) de champ électromagnétique et à partir de la position des capteurs (21, 22), et pour configurer le bras (14) articulé de sorte que toute partie métallique du bras (14) articulé se trouvant dans la zone de mesure (25) est située dans ladite région d'influence réduite (26), ladite région d'influence réduite (26) étant une région pour laquelle la présence d'une partie métallique du bras (14) articulé dans cette région perturbe de façon négligeable la détermination de la position de chacun des deux capteurs (21, 22).

9. Robot médical (10) selon la revendication 8 dans lequel le bras (14) articulé comporte un porte-outil (17) à une extrémité libre du bras (14) articulé, le porte-outil est réalisé dans un matériau amagnétique, et le deuxième capteur (22) est positionné au niveau du porte-outil (17).

10. Robot médical (10) selon la revendication 9 dans lequel le porte-outil (17) s'étend selon une direction longitudinale de la dernière section (18) du bras (14) articulé à l'extrémité libre du bras (14) articulé, le porte-outil (17) a une longueur au moins égale à 10 cm, et le deuxième capteur (22) est positionné à une extrémité distale du porte-outil (17).

11. Robot médical (10) selon l'une des revendication 8 à 10 dans lequel le générateur (23) de champ électromagnétique prend la forme d'une plaque, et la région d'influence réduite (26) est déterminée comme étant une région de la zone de mesure (25) située à l'opposé de ladite plaque par rapport à un plan (52) parallèle à la plaque et passant par le capteur (21, 22) qui est à la distance la plus grande de la plaque dans une direction orthogonale à la plaque.

12. Robot médical (10) selon l'une des revendication 8 à 10 dans lequel la région d'influence réduite (26) est déterminée comme étant une région de la zone de mesure (25) qui est située à l'opposé du générateur (23) de champ électromagnétique par rapport à un plan (53) orthogonal à une direction (52) passant par un centre (24) du générateur (23) de champ électromagnétique et par le capteur (21, 22) qui est à la plus grande distance du centre (24) du générateur (23) de champ électromagnétique.

13. Robot médical (10) selon l'une des revendications 8 à 12 dans lequel le bras (14) articulé est configuré en fonction d'un plan d'intervention mémorisé dans l'unité de contrôle (12) du robot médical (10), ledit plan d'intervention comportant des informations sur au moins une action à effectuer sur l'anatomie d'intérêt (31) du patient (30), la configuration du bras articulé étant déterminée à partir des positions des capteurs (21, 22) déterminées par le système de navigation pour permettre au praticien de réaliser ladite action.

14. Robot médical (10) selon la revendication 13 dans lequel une configuration du bras (14) articulé permettant d'effectuer une action du plan d'intervention est déterminée à partir :
- d'une image préopératoire comportant des informations sur ladite action,
- d'une image intra-opératoire comportant des informations sur la position de l'anatomie d'intérêt (31) du patient (30) par rapport à la position du premier capteur (21) situé au niveau de l'anatomie d'intérêt (31),
- d'un recalage de l'image intra-opératoire avec l'image préopératoire pour obtenir une image comportant à la fois les informations sur l'action planifiée à effectuer sur l'anatomie d'intérêt du patient (30) et les informations sur la position de ladite anatomie d'intérêt (31) par rapport à la position dudit premier capteur (21).

## Patentansprüche

1. Verfahren (100) zur Positionierung eines Gelenkarms (14) eines medizinischen Roboters (10), um einen praktizierenden Arzt bei einem medizinischen Eingriff an einer interessierenden Anatomie (31) eines Patienten (30) zu unterstützen, wobei der medizinische Roboter (10) eine Basis (11), mit der der Gelenkarm (14) verbunden ist, sowie eine Steuereinheit (12) enthält, die es ermöglicht, den Gelenkarm (14) in einer bestimmten Position zu konfigurieren, wobei der medizinische Roboter (10) von einem elektromagnetischen Navigationssystem unterstützt wird, das einen Generator (23) eines elektromagnetischen Felds und zwei elektromagnetische Sensoren (21, 22) enthält, wobei das erzeugte elektromagnetische Feld eine Messzone (25) bildet, in der die Position eines Sensors (21, 22) durch das elektromagnetische Navigationssystem bestimmt und dem medizinischen Roboter (10) mitgeteilt werden kann, wobei in einem Schritt vor dem Verfahren (100) ein erster Sensor (21) im Bereich der interessierenden Anatomie (31) positioniert wird, wobei ein zweiter Sensor (22) auf dem Gelenkarm (14) positioniert wird, wobei das Verfahren zur Positionierung des Gelenkarms (14) **dadurch gekennzeichnet ist, dass** das Verfahren enthält:
- eine Bestimmung (101), wenn die zwei Sensoren (21, 22) sich in der Messzone (25) befinden, eines Bereichs sogenannten "verringerten Einflusses" (26) der Messzone (25) ausgehend von der Position des Generators (23) eines elektromagnetischen Felds und ausgehend von der Position der Sensoren (21, 22), wobei der Bereich verringerten Einflusses (26) ein Bereich ist, in dem das Vorhandensein eines metallischen Teils des Gelenkarms (14) in diesem Bereich die Bestimmung der Position jedes der zwei Sensoren (21, 22) in vernachlässigbarer Weise beeinträchtigt,
- eine Konfiguration (102) des Gelenkarms (14) derart, dass jeder metallische Teil des Gelenkarms (14), der sich in der Messzone (25) befindet, innerhalb des Bereichs verringerten Einflusses (26) liegt,
wobei das Verfahren (100) der Positionierung des Gelenkarms (14) keinen Schritt der Ausführung eines medizinischen Handgriffs am Patienten enthält.

2. Verfahren (100) nach Anspruch 1, wobei der Generator (23) eines elektromagnetischen Felds die Form einer Platte annimmt, und der Bereich verringerten Einflusses (26) als ein Bereich der Messzone (25) bestimmt wird, der sich bezüglich einer Ebene (52) parallel zur Platte entgegengesetzt zur Platte befindet und durch den Sensor (21, 22) verläuft, der in einer Richtung orthogonal zur Platte im größten Abstand zur Platte ist.

3. Verfahren (100) nach Anspruch 1, wobei der Bereich verringerten Einflusses (26) als ein Bereich der Messzone (25) bestimmt wird, der sich entgegengesetzt zum Generator (23) eines elektromagnetischen Felds bezüglich einer Ebene (53) orthogonal zu einer Richtung (52) befindet, die durch eine Mitte (24) des Generators (23) eines elektromagnetischen Felds und durch den Sensor (21, 22) verläuft, der im größten Abstand zur Mitte (24) des Generators (23) eines elektromagnetischen Felds ist.

4. Verfahren (100) nach einem der Ansprüche 1 bis 3, wobei der Gelenkarm (14) abhängig von einem in der Steuereinheit (12) des medizinisches Roboters (10) gespeicherten Eingriffsplan konfiguriert wird, wobei der Eingriffsplan Informationen über mindestens eine an der interessierenden Anatomie (31) des Patienten (30) auszuführende Maßnahme enthält, wobei die Konfiguration des Gelenkarms ausgehend von den vom Navigationssystem bestimmten Positionen der Sensoren (21, 22) bestimmt wird, um es dem praktizierenden Arzt zu ermöglichen, die Maßnahme nach dem Verfahren (100) der Positionierung des Gelenkarms (14) durchzuführen.

5. Verfahren (100) nach Anspruch 4, wobei eine Konfiguration des Gelenkarms (14), die es ermöglicht, eine Maßnahme des Eingriffsplans auszuführen, bestimmt wird ausgehend von:
- einem präoperativen Bild, das Informationen über die Maßnahme enthält,
- einem intra-operativen Bild, das Informationen über die Position der interessierenden Anatomie (31) des Patienten (30) bezüglich der Position des ersten Sensors (21) enthält, der sich im Bereich der interessierenden Anatomie (31) befindet,
- einer Neueinstellung des intra-operativen Bilds mit dem präoperativen Bild, um ein Bild zu erhalten, das sowohl die Informationen über die an der interessierenden Anatomie des Patienten (30) auszuführende geplante Maßnahme als auch die Informationen über die Position der interessierenden Anatomie (31) bezüglich der Position des ersten Sensors (21) enthält.

6. Verfahren (100) nach Anspruch 5, wobei der erste Sensor (21) im intra-operativen Bild sichtbare Elemente enthält, und die Geometrie der Elemente bekannt ist.

7. Verfahren (100) nach einem der Ansprüche 1 bis 6, wobei die interessierende Anatomie (31) des Patienten (30) und der erste elektromagnetische Sensor (21) sich innerhalb des Körpers des Patienten (30) befinden.

8. Medizinischer Roboter (10) zur Unterstützung eines praktizierenden Arztes bei einem medizinischen Eingriff an einer interessierenden Anatomie (31) eines Patienten (30), wobei der medizinische Roboter (10) eine Basis (11), mit der ein Gelenkarm (14) verbunden ist, sowie eine Steuereinheit (12) enthält, die es ermöglicht, den Gelenkarm (14) in einer bestimmten Position zu konfigurieren, wobei der medizinische Roboter (10) dazu bestimmt ist, von einem elektromagnetischen Navigationssystem unterstützt zu werden, das einen Generator (23) eines elektromagnetischen Felds und zwei elektromagnetische Sensoren (21, 22) enthält, wobei das erzeugte elektromagnetische Feld eine Messzone (25) bildet, in der die Position eines Sensors (21, 22) durch das elektromagnetische Navigationssystem bestimmt und dem medizinischen Roboter (10) mitgeteilt werden kann, wobei ein erster Sensor (21) im Bereich der interessierenden Anatomie (31) positioniert ist, ein zweiter Sensor (22) auf dem Gelenkarm (14) positioniert ist, wobei der medizinische Roboter (10) **dadurch gekennzeichnet ist, dass** die Steuereinheit (12) konfiguriert ist, wenn die zwei Sensoren (21, 22) sich in der Messzone (25) befinden, einen Bereich sogenannten "verringerten Einflusses" (26) der Messzone (25) ausgehend von der Position des Generators (23) eines elektromagnetischen Felds und ausgehend von der Position der Sensoren (21, 22) zu bestimmen, und um den Gelenkarm (14) so zu konfigurieren, dass jeder metallische Teil des Gelenkarms (14), der sich in der Messzone (25) befindet, im Bereich verringerten Einflusses (26) liegt, wobei der Bereich verringerten Einflusses (26) ein Bereich ist, in dem das Vorhandensein eines metallischen Teils des Gelenkarms (14) in diesem Bereich die Bestimmung der Position jedes der zwei Sensoren (21, 22) vernachlässigbar beeinträchtigt.

9. Medizinischer Roboter (10) nach Anspruch 8, wobei der Gelenkarm (14) an einem freien Ende des Gelenkarms (14) einen Werkzeugträger (17) enthält, der Werkzeugträger aus einem nicht magnetischen Material hergestellt ist, und der zweite Sensor (22) im Bereich des Werkzeugträgers (17) positioniert ist.

10. Medizinischer Roboter (10) nach Anspruch 9, wobei der Werkzeugträger (17) sich vom letzten Abschnitt (18) des Gelenkarms (14) am freien Ende des Gelenkarms (14) gemäß einer Längsrichtung erstreckt, der Werkzeugträger (17) eine Länge mindestens gleich 10 cm hat, und der zweite Sensor (22) an einem distalen Ende des Werkzeugträgers (17) positioniert ist.

11. Medizinischer Roboter (10) nach einem der Ansprüche 8 bis 10, wobei der Generator (23) eines elektromagnetischen Felds die Form einer Platte annimmt, und der Bereich verringerten Einflusses (26) als ein Bereich der Messzone (25) bestimmt wird, der sich entgegengesetzt zur Platte bezüglich einer Ebene (52) parallel zur Platte und durch den Sensor (21, 22) verlaufend befindet, der in einer Richtung orthogonal zur Platte im größten Abstand zur Platte ist.

12. Medizinischer Roboter (10) nach einem der Ansprüche 8 bis 10, wobei der Bereich verringerten Einflusses (26) als ein Bereich der Messzone (25) bestimmt wird, der sich entgegengesetzt zum Generator (23) eines elektromagnetischen Felds bezüglich einer Ebene (53) orthogonal zu einer Richtung (52) befindet, die durch eine Mitte (24) des Generators (23) eines elektromagnetischen Felds und durch den Sensor (21, 22) verläuft, der im größten Abstand zur Mitte (24) des Generators (23) eines elektromagnetischen Felds ist.

13. Medizinische Roboter (10) nach einem der Ansprüche 8 bis 12, wobei der Gelenkarm (14) abhängig von einem in der Steuereinheit (12) des medizinisches Roboters (10) gespeicherten Eingriffsplan konfiguriert wird, wobei der Eingriffsplan Informationen über mindestens eine an der interessierenden Anatomie (31) des Patienten (30) auszuführende Maßnahme enthält, wobei die Konfiguration des Gelenkarms ausgehend von den vom Navigationssystem bestimmten Positionen der Sensoren (21, 22) bestimmt wird, um es dem praktizierenden Arzt zu erlauben, die Maßnahme durchzuführen.

14. Medizinische Roboter (10) nach Anspruch 13, wobei eine Konfiguration des Gelenkarms (14), die es ermöglicht, eine Maßnahme des Eingriffsplans auszuführen, bestimmt wird ausgehend von:
- einem präoperativen Bild, das Informationen über die Maßnahme enthält,
- einem intra-operativen Bild, das Informationen über die Position der interessierenden Anatomie (31) des Patienten (30) bezüglich der Position des ersten Sensors (21) enthält, der sich im Bereich der interessierenden Anatomie (31) befindet,
- einer Neueinstellung des intra-operativen Bilds mit dem präoperativen Bild, um ein Bild zu erhalten, das sowohl die Informationen über die an der interessierenden Anatomie des Patienten (30) auszuführende geplante Maßnahme als auch die Informationen über die Position der interessierenden Anatomie (31) bezüglich der Position des ersten Sensors (21) enthält.

## Claims

1. Method (100) for positioning an articulated arm (14) of a medical robot (10) to assist a practitioner in a medical operation on an anatomical location of interest (31) of a patient (30), the medical robot (10) comprising a base (11) to which the articulated arm (14) is linked, and a control unit (12) allowing the articulated arm (14) to be configured in a determined position, the medical robot (10) being assisted by an electromagnetic navigation system comprising an electromagnetic field generator (23) and two electromagnetic sensors (21, 22), the generated electromagnetic field forming a measurement zone (25) in which the position of a sensor (21, 22) can be determined by the electromagnetic navigation system and communicated to the medical robot (10), a first sensor (21) being positioned, in a step prior to said method (100) at the anatomical location of interest (31), a second sensor (22) being positioned on the articulated arm (14), the method for positioning the articulated arm (14) being **characterized in that** said method comprises:
- a determination (101), when the two sensors (21, 22) are located in the measurement zone (25), of a so-called "region of reduced influence" (26) of the measurement zone (25) from the position of the electromagnetic field generator (23) and from the position of the sensors (21, 22), said region of reduced influence (26) being a region for which the presence of a metal part of the articulated arm (14) in this region negligibly disturbs the determination of the position of each of the two sensors (21, 22),
- a configuration (102) of the articulated arm (14) such that any metal part of the articulated arm (14) located in the measurement zone (25) is situated within the region of reduced influence (26),
said method (100) for positioning the articulated arm (14) not including a step of execution of a medical act on the patient.

2. Method (100) according to Claim 1, wherein the electromagnetic field generator (23) takes the form of a plate, and the region of reduced influence (26) is determined as being a region of the measurement zone (25) situated opposite said plate with respect to a plane (52) parallel to the plate and passing through the sensor (21, 22) which is at the greatest distance from the plate in a direction orthogonal to the plate.

3. Method (100) according to Claim 1, wherein the region of reduced influence (26) is determined as being a region of the measurement zone (25) which is situated opposite the electromagnetic field generator (23) with respect to a plane (53) orthogonal to a direction (52) passing through a center (24) of the electromagnetic field generator (23) and through the sensor (21, 22) which is at the greatest distance from the center (24) of the electromagnetic field generator (23).

4. Method (100) according to one of Claims 1 to 3, wherein the articulated arm (14) is configured according to an operation plan stored in the control unit (12) of the medical robot (10), said operation plan comprising information on at least one action to be performed on the anatomical location of interest (31) of the patient (30), the configuration of the articulated arm being determined from the positions of the sensors (21, 22) determined by the navigation system to allow the practitioner to perform said action subsequent to the method (100) for positioning the articulated arm (14).

5. Method (100) according to Claim 4, wherein a configuration of the articulated arm (14) allowing an action of the operation plan to be performed is determined from:
- a preoperative image comprising information on said action,
- an intra-operative image comprising information on the position of the anatomical location of interest (31) of the patient (30) with respect to the position of the first sensor (21) situated at the anatomical location of interest (31),
- a registration of the intra-operative image with the preoperative image to obtain an image comprising both the information on the planned action to be performed on the anatomical location of interest of the patient (30) and the information on the position of said anatomical location of interest (31) with respect to the position of said first sensor (21).

6. Method (100) according to Claim 5, wherein the first sensor (21) comprises elements visible in said intra-operative image, and the geometry of said elements is known.

7. Method (100) according to one of Claims 1 to 6, wherein the anatomical location of interest (31) of the patient (30) and the first electromagnetic sensor (21) are situated inside the body of the patient (30).

8. Medical robot (10) for assisting a practitioner in a medical operation on an anatomical location of interest (31) of a patient (30), the medical robot (10) comprising a base (11) to which an articulated arm (14) is linked, and a control unit (12) allowing the articulated arm (14) to be configured in a determined position, the medical robot (10) being intended to be assisted by an electromagnetic navigation system comprising an electromagnetic field generator (23) and two electromagnetic sensors (21, 22), the generated electromagnetic field forming a measurement zone (25) in which the position of a sensor (21, 22) can be determined by the electromagnetic navigation system and communicated to the medical robot (10), a first sensor (21) being positioned at the anatomical location of interest (31), a second sensor (22) being positioned on the articulated arm (14), the medical robot (10) being **characterized in that** the control unit (12) is configured, when the two sensors (21, 22) are located in the measurement zone (25), to determine a so-called "region of reduced influence" (26) of the measurement zone (25) from the position of the electromagnetic field generator (23) and from the position of the sensors (21, 22), and to configure the articulated arm (14) such that any metal part of the articulated arm (14) located in the measurement zone (25) is situated within said region of reduced influence (26), said region of reduced influence (26) being a region for which the presence of a metal part of the articulated arm (14) in this region negligibly disturbs the determination of the position of each of the two sensors (21, 22).

9. Medical robot (10) according to Claim 8, wherein the articulated arm (14) comprises a tool-holder (17) at a free end of the articulated arm (14), the tool-holder is produced in a non-magnetic material, and the second sensor (22) is positioned on the tool-holder (17).

10. Medical robot (10) according to Claim 9, wherein the tool-holder (17) extends in a longitudinal direction of the last section (18) of the articulated arm (14) at the free end of the articulated arm (14), the tool-holder (17) has a length at least equal to 10 cm, and the second sensor (22) is positioned at a distal end of the tool-holder (17).

11. Medical robot (10) according to one of Claims 8 to 10, wherein the electromagnetic field generator (23) takes the form of a plate, and the region of reduced influence (26) is determined as being a region of the measurement zone (25) situated opposite said plate with respect to a plane (52) parallel to the plate and passing through the sensor (21, 22) which is at the greatest distance from the plate in a direction orthogonal to the plate.

12. Medical robot (10) according to one of Claims 8 to 10, wherein the region of reduced influence (26) is determined as being a region of the measurement zone (25) which is situated opposite the electromagnetic field generator (23) with respect to a plane (53) orthogonal to a direction (52) passing through a center (24) of the electromagnetic field generator (23) and through the sensor (21, 22) which is at the greatest distance from the center (24) of the electromagnetic field generator (23) .

13. Medical robot (10) according to one of Claims 8 to 12, wherein the articulated arm (14) is configured according to an operation plan stored in the control unit (12) of the medical robot (10), said operation plan comprising information on at least one action to be performed on the anatomical location of interest (31) of the patient (30), the configuration of the articulated arm being determined from the positions of the sensors (21, 22) determined by the navigation system to allow the practitioner to perform said action.

14. Medical robot (10) according to Claim 13, wherein a configuration of the articulated arm (14) allowing an action of the operation plan to be performed is determined from:
- a preoperative image comprising information on said action,
- an intra-operative image comprising information on the position of the anatomical location of interest (31) of the patient (30) with respect to the position of the first sensor (21) situated at the anatomical location of interest (31),
- a registration of the intra-operative image with the preoperative image to obtain an image comprising both the information on the planned action to be performed on the anatomical location of interest of the patient (30) and the information on the position of said anatomical location of interest (31) with respect to the position of said first sensor (21).
